# EUROPÄISCHE PATENTANMELDUNG

(11) **EP 0 713 881 A2**
(43) Veröffentlichungstag der Anmeldung: **29.05.1996**
(21) Anmeldenummer: 95114846.9
(22) Anmeldetag: 21.09.1995
(51) Int. Cl.: C07K 14/795, A61K 38/41

(54) **Einsatz von vWF-enthaltenden Konzentraten als Kombinationstherapie bei der Therapie mit Antithrombotika und Fibrinolytika**

(30) Priorität: 20.10.1994 DE 4437544
(71) Anmelder: BEHRINGWERKE Aktiengesellschaft, 35001 Marburg (DE)
(72) Erfinder: Reers, Martin, Dr., D-35041 Marburg (DE); Dickneite, Gerhard, Dr., D-35043 Marburg (DE)

(57) **Zusammenfassung**

Die Erfindung betrifft die Gabe von von Willebrand Faktor (vWF)-enthaltenden Medikamenten beim therapeutischen Einsatz von Blutantikoagulantien allein, oder in Verbindung mit Fibrinolytika. Die Gabe kann sowohl gleichzeitig als auch nachfolgend der antikoagulierenden und lysierenden Behandlung zur Reduzierung der Blutungsrisiken bei Patienten verabreicht werden. Es wird ein Therapieprinzip beschrieben, welches die gewünschte Wirkung der Antikoagulation und oder Fibrinolyse von der unerwünschten Nebenwirkung der Blutungsneigung während der Therapie abtrennt.

## Beschreibung

Die Erfindung betrifft die Gabe von von Willebrand Faktor (vWF)-enthaltenden Medikamenten beim therapeutischen Einsatz von Blutantikoagulantien allein, oder in Verbindung mit Fibrinolytika. Die Gabe kann sowohl gleichzeitig als auch nachfolgend der antikoagulierenden und lysierenden Behandlung zur Reduzierung der Blutungsrisiken bei Patienten verabreicht werden. Es wird ein Therapieprinzip beschrieben, welches die gewünschte Wirkung der Antikoagulation und oder Fibrinolyse von der unerwünschten Nebenwirkung der Blutungsneigung während der Therapie abtrennt.

Ungleichgewichte der Komponenten der Gerinnung und der Fibrinolyse manifestieren sich klinisch zum einen in Thromboseneigungen und zum anderen in Blutungsneigungen. Beide pathologischen Zustände können sich lebensbedrohlich auswirken. Im Falle der Thromboseneigung, wie z.B. beim akuten Myokardinfarkt, wird in vielen Fällen versucht, regulierend in das aktuelle Lyse- und Gerinnungsungleichgewicht einzugreifen. Das fibrinolytische System wird z.B. durch Gaben von Streptokinase (SK) oder Plasminogen-Activatoren (t-PA, uPA, ggf. rekombinant) unterstützt, um bereits bestehende Blutgerinnsel aufzulösen. Das Gerinnungssystem wird z.B. durch Heparin, "low molecular weight heparin" (LMWH), oder durch Thrombininhibitoren wie z.B. den synthetischen Inhibitor MCI-9038 (Tamao Y et al. Thromb. Haemost. 56, 1, 28-34, 1987) oder wie rekombinantes Hirudin (r-Hir), oder durch Faktor Xa-Inhibitoren wie rekombinantes "tick anticoagulant protein" (r-TAP), oder Plättchenantagonisten wie Aspirin, 7E3-Antikörper gegen Thrombozyten vollständig unterdrückt bzw gedämpft. Dadurch wird eine Öffnung der thrombotisch verschlossenen Gefäße erreicht und die erneute Bildung eines Thrombus vermieden. Allerdings ist ein gewisses Maß Thrombusbildungsfähigkeit bei Gefäßläsionen notwendig, um Blutungen an diesen Stellen zu verhindern.

Gemäß des Standes der Technik versucht man, möglicherweise auftretenden, lebensbedrohenden Blutungen bei antikoagulierender oder in Kombination lysierender Therapie durch Abbrechen der Therapie und durch Applikation von Koagulationsförderern (Antidote) entgegenzuwirken. Solche Koagulationsförderer beinhalten Faktor VIII (FVIII) oder Substanzen, die die endogene Konzentration von FVIII im Blut erhöhen, wie Desmopressin (DDAVP) (Mannucci PM, Ruggeri ZM, Pareti FI, Capitano A, 1977, Lancet, 1, 869-872; EP 0 367 713 B1, 1992; US Patent 5,204,323, 1993) oder ein Antifibrinolytikum wie Aprotinin in einer Mischung mit Desmopressin, Tranexamsäure, ε-Aminocapronsäure und 4-Aminomethylbenzoesäure (WO 9220361, 1992). Andere Koagulationsförderer bestehen unter anderem aus teilweise bereits aktivierten Gerinnungsfaktoren wie in dem Handelsprodukt FEIBA® (Immuno AG), oder aus einem Prothrombinkonzentrat wie in dem Handelsprodukt Beripex® (Behringwerke AG) oder in dem Handelsprodukt Autoplex® (Baxter Inc.). All diese Antidote bewirken, daß in erster Linie die therapeutischen Wirkungen der Wirkstoffe und dadurch auch die wirkstoffbedingten Nebenwirkungen reduziert werden. Die bei vorgenannten Antidoten antidotbedingte Wirkungen bergen aber die Gefahr, daß das vorhergehende Therapieergebnis (die Antikoagulation) zunichte gemacht wird und demzufolge ein großes Thromboserisiko besteht.

Ein quantitatives Maß für die antikoagulierende und fibrinolytische Wirkung in der Klinik ist die aktivierte partielle Thromboplastinzeit (aPTT), wobei die Blutungszeit (BT) ein Maß für die Blutungsneigung ist. Eine auch klinisch im Menschen gut reproduzierbare Methode zur Bestimmung der Blutungszeit ist die Messung der Hautblutungszeit nach der Simplate-Methode (Lethagen S. und Kling S, 1993, Thrombosis Haemostasis 70, 595-597). aPTT und BT korrelieren im allgemeinen recht gut miteinander in der Form, daß eine erhöhte Wirkung gemessen in erhöhten aPTT-Werten, auch erhöhte Nebenwirkungen, d.h. längere Blutungszeiten nach sich zieht.

Aus mechanistischen Untersuchungen zum Ablauf der Blutstillung nach einer Gefäßläsion ergibt sich, daß die Blutplättchen in der Phase der Primärhämostase in der Hauptsache an subendotheliale Kollagenfasern mit Hilfe des von Willebrand Faktors (vWF) binden. vWF ist der einzige Faktor, der in der Lage ist, effizient sowohl bei niedrigen (z.B. im venösen Bereich) als auch bei hohen Scherraten (z.B. im arteriellen, koronaren Bereich oder bei Plaque-bedingten Verengungen der Gefäße) Blutplättchen an das exponierte Kollagen zu binden (Ruggeri ZM, Seminars in Hematology, 1994, 31, 229-239). Eine nachfolgende Plättchenaggregation und eine Retraktion und Kontraktion der aggregierten Plättchen durch Mitwirkung von Thrombin bildet in der Sekundärhämostase einen blutstillenden Verschluß (Hemker HC, und Poliwoda H, 1993, 1-18, Barthels M und Polidowa H, Herausgeber, Thieme Verlag, Stuttgart, Deutschland).

vWF ist das größte z.Z. bekannte lösliche Plasmaprotein. Es ist ein multimeres Glykoprotein mit zwei biologischen Eigenschaften. Es vermittelt bei lokalen Gefäßverletzungen die Plättchenadhäsion mit nachfolgender Thrombusbildung und es dient als "Carrier" für den prokoagulatorischen Gerinnungsfaktor VIII (Ruggeri ZM. 1993 Current Opinion in Cell Biology, 5, 898-906). vWF kommt in gewissen Mengen in Subendothel vor und wird ebenfalls in Factor VIII-freier Form in den α-Granula der Blutplättchen gespeichert. Blutplättchen besitzen zwei Rezeptoren für vWF: 1. GP Ib im GP Ib-IX-V Komplex und 2. GP IIb-IIIa (Ruggeri ZM, 1994 Seminars in Hematology 31, 229-239). vWF vermittelt über den ersteren Rezeptor eine Plättchenadhäsion an der Stelle der Gefäßverletzung, wobei nachfolgend vWF mit dem GP IIb-IIIa Rezeptor die nachfolgende Plättchenaggregation unterstützt, welche allerdings in der Hauptsache durch die Fibrinogenbindung an den GPIIb-IIIa Rezeptor getragen wird. Mit diesem Hintergrund wird in der Literatur diskutiert, Inhibitoren für die Bindung von vWF als Prinzip für ein Antikoagulans zu verwenden (Alevriadou BR, Moake JL, Turner NA, Ruggeri ZM, Folie BJ, Philips MD, Schreiber AB, Hrinda ME, McIntire IV, 1993, Blood, 81, 1263-1276; Gralnick HR, Williams S, McKeown L, Kramer W, Krutzsch H, Gorecki M, Pinet A Garfinkel LI, 1992 Proc. Natl. Acad. Sci. USA, 89, 7880-7884).

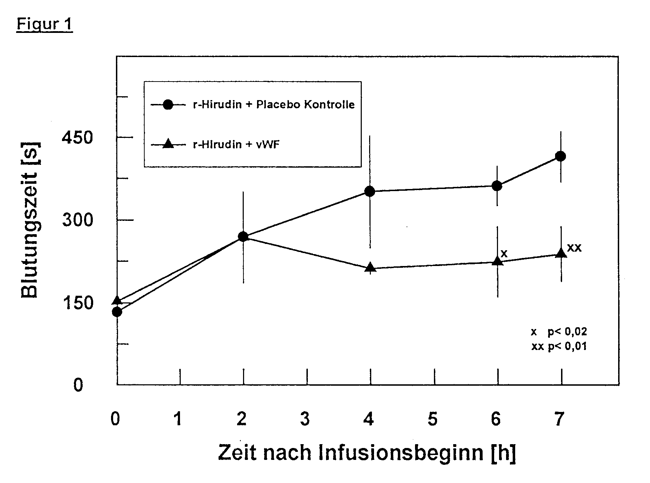

Überraschenderweiser wurde gefunden, daß im Schwein bei einem antikoagulatorischen Therapieprotokoll mit z.B. r-Hirudin eine zusätzliche intravenöse Gabe von Plasma vWF die antikoagulatorische systemische Wirkung (aPTT) nicht beinträchtigte, wohingegen aber die Blutungsnebenwirkung (BT) auf ein Niveau unterhalb der zweifachen Blutungszeitverlängerung abnahm und dann konstant blieb. Im Kontrollexperiment ohne vWF Infusion war die BT auf etwa das 3-fache der normalen Blutungszeit angestiegen (Fig. 1).

Beschreibung der Figur:
- Fig. 1:: Wirkung von vWF auf die durch r-Hirudin induzierte Hautblutungszeit im Schwein 0,3 mg/kg x h r-Hirudin (Placebo-Gruppe,●); 0,3 mg/kg x h r-Hirudin plus nach 3 Stunden: 66 vWF-Einheiten/kg i.v. Bolus und 187 vWF-Einheiten/kg x h i.v. Infusion vWF (vWF-Gruppe,▲)
Die Lösung der Aufgabe der Erfindung besteht somit in einem Verfahren bei der antikoagulatorischen Therapie und gegebenenfalls in Kombination mit einer fibrinolytischen Therapie, welches durch geeignete Supplementierung mit einer vWF-enthaltenden Lösung eine Trennung der oben beschriebenen Korrelation zwischen der erwünschten Wirkung (angezeigt durch aPTT) und der unerwünschten Nebenwirkung der Blutung (angezeigt durch BT) bewirkt: die therapeutische Wirkung bleibt erhalten, d. h. auch ein Thromboserisiko bleibt ausgeschlossen, aber die durch die Wirkung der Antikoagulantien oder Fibrinolytika hervorgerufenen Nebenwirkungen werden minimiert.

Die Erfindung betrifft deshalb die Verwendung von vWF zur Herstellung eines als Pseudo-Antidot wirkenden Mittels bei durch Gabe von antithrombotischen und/oder fibrinolytischen Mitteln erzeugten Blutungen. Des weiteren betrifft die Erfindung pharmazeutische Zusammensetzungen, die als Komponente A ein antithrombotisches Mittel und/oder ein fibrinolytisches Mittel enthalten und als Komponente B vWF oder Fragmente mit vWF-Aktivität enthalten.

Das eingesetzte vWF-Konzentrat kann z.B. das Handelsprodukt Haemate® HS (Behringwerke AG), das neben vWF auch FVIII enthält, sein.

### Beispiel 1:

### Verlauf der Blutungszeit und der aPTT nach Hirudin-Infusion und nach Ko-infusion von vWF-Lösung (Haemate® HS äquivalent zu Haemate P®).

Insgesamt 7 männlichen kastrierten Schweinen wurde über 7 Stunden eine intravenöse Infusion von 0.3 mg/kg x h rekombinantem Hirudin (r-Hirudin) verabreicht. Drei Stunden nach Infusionsbeginn wurden 3 Tiere zusätzlich mit einem i.v. Bolus eines vWF-enthaltenden Konzentrates (66 vWF-Einheiten/kg), gefolgt von einer zweistündigen Infusion von 187 vWF-Einheiten/kg x h (vWF-Gruppe) behandelt. Vier Tiere bekamen statt vWF ein entsprechendes Volumen an Kochsalzlösung appliziert (Placebo-Gruppe). Während des Versuches wurde die Hautblutungszeit mit der Simplate®-Methode (Organon-Teknika) bestimmt und die aPTT im Plasma entsprechend der Neothromtin®-Methode (Behringwerke AG) gemessen. Wie Abb. 1 zeigt, steigt die Blutungszeit zunächst in beiden Gruppen an, in der Placebo-Gruppe steigt die Blutungszeit nach 7 Stunden auf das ca. 3-fache des Normalwertes an. In der vWF-Gruppe nimmt die Blutungszeit nach der Behandlung durch vWF ab, um nach 7 Stunden fast den Normalwert wieder zu erreichen (statistisch signifikante Unterschiede bei 6 und 7 Stunden zwischen beiden Gruppen). vWF ist somit in der Lage, das durch den Thrombininhibitor r-Hirudin erhöhte Blutungsrisiko wieder zu normalisieren.

Als Maß für die antikoagulatorische Aktivität von r-Hirudin wurde die aPTT im gleichen Experiment gemessen. Wie Tab. 1 zeigt, ließ sich zwischen der Placebo- und der vWF-Gruppe kein signifikanter Unterschied ermitteln, in beiden Gruppen wurde eine ca. 2-fache Erhöhung der aPTT beobachtet.

**Tabelle 1**

| **Zeit nach Infusionsbeginn [h]** | **aPTT [s]** | |
|---|---|---|
| | **Placebo-Gruppe** | **vWF-Gruppe** |
| 0 | 124,3 ± 56,0 | 101,0 ± 34,3 |
| 6 | 185,7 ± 103,2 | 213,7 ± 80,8 |
| 7 | 165,0 ± 83,7 | 214,8 ± 32,3 |

Als Antikoagulantien, die mit dem vWF kombiniert werden können, sind zu nennen: Heparin; LMW-Heparin; synthetische (rekombinante) Thrombininhibitoren wie r-Hir, deren Derivate wie Polyethylenglykolhirudin oder Hirulog; synthetische niedermolekulare Thrombininhibitoren wie Argartroban (MCI 9098, siehe Tamao Y, et al. a.a.O.; synthetische oder rekombinante FXa Inhibitoren wie TAP; synthetische oder rekombinante FVII Inhibitoren wie tissue factor pathway inhibitor (TFPI); Blutplättchenantagonisten wie Acetylsalicylsäure oder synthetische Fibrinogenrezeptor-Inhibitoren, Thrombozyten-Antikörper (z.B. 7E3); Vitamin K Antagonisten wie Warfarin, Phenprocoumon, Acenocoumarol.

Folgende Fibrinolytika sind mit den vorstehenden Antikoagulantien und vWF zur Kombination gut geeignet: Streptokinase; Plasminogen-Aktivatoren (rt-PA, uPA) und deren Derivate, wie z.B. Apsac.

### Beispiel 2

### Wirkung einer vWF-Lösung (Haemate®) auf die Blutungszeit bei einer Kombination von Hirudin und Aspirin

In diesem Experiment wurde bei insgesamt 4 Schweinen der Thrombininhibitor Hirudin mit dem Blutplättcheninhibitor Aspirin kombiniert. Aspirin wurde vor der Hirudininfusion über eine halbe Stunde in einer Konzentration von 20 mg/kg und Hirudin in einer Konzentration von 0.3 mg/kg x h über 7 Stunden (t = 0) infundiert.

Nach 3 Stunden erhielten 2 Tiere vWF-Lösung (Haemate® HS) und zwar 66 vWF-Einheiten/kg als i. v. Bolus und danach eine 2-Stunden-i. v. Infusion von 187 vWF-Einheiten/kg x h. Die anderen Tiere erhielten eine entsprechende Menge an Kochsalzlösung. Tabelle 2 zeigt, daß die Blutungszeit nach 3 Stunden auf das ca. 2.5 fache des Ausgangswertes (O-Wert) angestiegen war. Die Gabe von vWF-Lösung führte zu einer deutlichen Abnahme der Blutungszeit (auf das 1.8 fache) nach 6 Stunden, während bei den, NaCl-behandelten Tieren die Blutungszeit weiter auf das 2.9 fache anstieg.

**Tabelle 2**

| Antikoagulation durch Hirudin und Aspirin: Reduktion der Blutungszeit durch vWF-Konzentrat | | | |
|---|---|---|---|
| | ***Blutungszeit** (**Vielfaches des O-Wertes**)* | | |
| ***Behandlung*** | ***O-Wert*** | ***3 Stunden*** | ***6 Stunden*** |
| Antikoagulation (Hirudin + Aspirin + NaCl | 1 | 2.5 ± 0.4 | 2.9 ± 0.4 |
| Antikoagulation (Hirudin + Aspirin + vWF)* | | | 1.8 ± 0.5 |

| | | | |
|---|---|---|---|
| * Haemate® HS | | | |

### Beispiel 3

Da die verwendete vWF-Lösung (Haemate® HS) außer vWF noch den Gerinnungsfaktor VIII (F VIII) enthielt, wurde geprüft, ob die Gabe eines F VIII-armen vWF-Konzentrates ebenfalls zu Reduktion der r-Hirudin induzierten Blutungszeitverlängerung genutzt werden kann. 7 Schweinen wurde über 7 Stunden 0.3 mg/kg x h r-Hirudin infundiert. Nach 3 Stunden wurden 3 Schweine mit einem F VIII-armen vWF-Konzentrat (66 vWF Einheiten/kg als i. v. Bolus, gefolgt von 187 vWF Einheiten/kg x h als i. v. Infusion) behandelt. 4 Schweine erhielten Kochsalzlösung in der entsprechenden Menge. Tab. 3 zeigt, daß auch das F VIII-arme vWF-Konzentrat die durch r-Hirudin angestiegene Blutungszeit reduziert.

**Tabelle 3**

| Wirkung eines Faktor VIII-armen vWF-Konzentrates auf die r-Hirudin induzierte Hautblutungszeit | | | |
|---|---|---|---|
| | ***Blutungszeit (Vielfaches des O-Wertes)*** | | |
| ***Behandlung*** | ***O-Wert*** | ***3 Stunden*** | ***6 Stunden*** |
| r-Hirudin + NaCl | 1 | 2.6 ± 1.0 | 2.9 ± 0.75 |
| r-Hirudin + vWF | | | 1.9 ± 0.5 |

## Patentansprüche

1. Verwendung von vWF oder Fragmenten mit vWF-Aktivität zur Herstellung eines als Antidot wirkenden Mittels bei durch Gabe von antithrombotischen und/oder fibrinolytischen Mitteln erzeugten Blutungen.

2. Pharmazeutische Zusammensetzung, die als Komponente A ein antithrombotisches Mittel und/oder ein fibrinolytisches Mittel und als Komponente B vWF oder Fragmente mit vWF-Aktivität enthält.

3. Pharmazeutische Zusammensetzung nach Anspruch 2, dadurch gekennzeichnet, daß die Einzelkomponenten in getrennten Einheitsdosierungsformen vorliegen.
